**Europäisches Patentamt**

**European Patent Office**

**Office européen des brevets**

(19)

(11) Veröffentlichungsnummer: **0 135 804**
A2

(12) **EUROPÄISCHE PATENTANMELDUNG**

(21) Anmeldenummer: **84110026.6**

(22) Anmeldetag: **22.08.84**

(51) Int. Cl.⁴: **A 61 B 17/58**

(30) Priorität: **09.09.83 DE 3332642**

(43) Veröffentlichungstag der Anmeldung: **03.04.85** Patentblatt 85/14

(84) Benannte Vertragsstaaten: **AT BE CH FR GB LI LU NL SE**

(71) Anmelder: **ORTOPEDIA GMBH, Salzredder, D-Kiel 14 (DE)**

(72) Erfinder: **Brudermann, Uwe, Dr., c/o ORTOPEDIA GMBH Salzredder, D-2300 Kiel 14 (DE)**

(74) Vertreter: **Patentanwälte Henkel, Pfenning, Feiler, Hänzel & Meinig, Möhlstrasse 37, D-8000 München 80 (DE)**

(54) **Vorrichtung zum Auffinden von Querbohrungen intramedullärer Implantate.**

(57) Die Vorrichtung dient zum Auffinden von Querbohrungen am distalen Ende von implantierten Verriegelungsnägeln bei der Frakturbehandlung von Knochen. Die Vorrichtung enthält zumindest einen ein axialsymmetrisches Feld erzeugenden Magneten (13, 16) und eine Magnetfeldmeßeinrichtung (8, 9) mit axialer Empfangscharakteristik, wobei eines dieser beiden Richtelemente in der Achse der zu lokalisierenden Querbohrung (11) ausrichtbar ist. Das andere der beiden Richtelemente, beispielsweise der Magnet (13), ist außerhalb frei beweglich und kann somit zur Achse der Querbohrung ausgerichtet werden. Die Ausgangssignale der Magnetfeldmeßeinrichtung (8, 9) werden über eine Signalauswerteeinrichtung (15) in einer Anzeigeeinrichtung (17) dargestellt. Durch relative Verschiebung des Magneten (13) und der Magnetfeldmeßeinrichtung (8, 9) können deren beide Achsen zur Deckung gebracht werden, was in der Anzeigeeinrichtung durch eine Nullpunktanzeige darstellbar ist.

Die Erfindung betrifft eine Vorrichtung zum Auffinden von Querbohrungen intramedullärer Implantate bei der Frakturbehandlung von Knochen, insbesondere zum Auffinden von Querbohrungen am distalen (dem Körper abgewandten) Ende von implantierten Verriegelungsnägeln, und zur koaxialen Ausrichtung einer Bohrvorrichtung zu diesen Querbohrungen.

Knochennägel werden zur gegenseitigen intramedullären Fixierung von Fragmenten der langen Röhrenknochen bei deren Frakturbehandlung eingesetzt. Eine Sonderausführung dieser Knochennägel besitzt proximal, d.h. dem Körper zugewandt, wie auch distal jeweils senkrecht zur Längsachse des Nagels angeordnete Bohrungen, die dazu dienen, nach dem Einbringen dieser Nägel über zusätzliche Schrauben oder Stifte die gelenknahen Knochenfragmente starr mit dem Nagel zu verbinden und so deren gegenseitige Position formschlüssig gegeneinander zu verriegeln. Das ist insbesondere dann angezeigt, wenn es sich um Mehrfachbrüche, Trümmerbrüche oder Brüche mit Defektstrecken handelt und eine Versorgung über externe, d.h. außerhalb der Gliedmaßen angeordnete Fixationselemente nicht notwendig oder nicht angezeigt ist.

Da der Markraumkanal der langen Röhrenknochen im allgemeinen weder über seine Gesamtlänge einen gleichbleibenden Querschnitt aufweist noch geradlinig ist, werden zur Knochennagelung meist Nägel mit solchen Profilformen eingesetzt, die eine mehr oder weniger ausgeprägte geometrische Anpassung des Nagels an die Form des Markraumkanals beim Eintreiben der Nägel zulassen. Die Folge ist, daß sich diese Nägel beim Eintreiben im allgemeinen sowohl verwinden als auch verbiegen. Nach dem Eintreiben ist deshalb die genaue Lage des distalen Endes des Nagels - wenn dieser in üblicher Weise vom proximalen Ende aus eingetrieben wurde - vom proximalen Ende aus nicht präzise bestimmbar.

0135804

Aus diesem Grund ist auch nicht voraussehbar, welche genaue Lage die distalen Bohrungen des Nagels beim Vorgang des Eintreibens einnehmen werden und wo demnach die Bohrungen im distalen Fragment des Knochens anzubringen sind, um dieses mit dem quer zur Nagel- bzw. Knochenlängsachse mit Hilfe der erwähnten Schrauben oder Stifte zu verriegeln. Diese Bohrungen sind jedoch mit großer Genauigkeit in den Knochen einzubringen, um zu verhindern, daß beim Bohrvorgang metallische Bohrspäne vom Nagelmaterial an den Rändern der Querbohrungen im Nagel abgetragen werden. Solche Bohrspäne können sonst im intramedullären Bereich des Knochens verbleiben und dort den Heilungsverlauf wesentlich negativ beeinträchtigen. Aus diesem Grund muß auch ein gemeinsames Bohren der distalen Löcher in Knochen und Nagel ausgeschlossen werden.

Zur Auffinden der distalen Löcher existieren verschiedene Verfahren und Vorrichtungen, die üblicherweise als "distale Zielgeräte" bekannt sind. Ihre Arbeitsweise soll nachfolgend kurz erörtert werden:

1. Für das Auffinden der proximalen Bohrungen wird ein mechanisches Zielgerät verwendet, das am proximalen Nagelende mechanisch fixiert wird. Dieses Zielgerät enthält eine Bohrlehre, die mit ausreichender Genauigkeit koaxial mit der jeweils nur wenige Zentimeter vom proximalen Nagelende entfernten proximalen Bohrung des Nagels fluchtet, selbst wenn eine Deformation des Nagels beim Eintreiben stattgefunden hat. Alle bekanntgewordenen Versuche, nach diesem Verfahren arbeitende Zielgeräte auch für die Auffindung der distalen Löcher einzusetzen, sind bislang gescheitert, sofern dabei Nägel eingesetzt wurden, die der ursprünglichen Forderung entsprechend ausreichend nachgiebig waren, um sich beim Eintreiben der Form des Markraumkanals anzupassen.

2. Für den Einsatz mit Knochennägeln vom kreisringförmigen Querschnitt besteht allein die Gefahr der
Verbiegung des Nagels, während Rotationsfehler
praktisch ausgeschlossen werden können. Hierbei
genügt es daher, die genaue Lage des Nagelendes
zu ermitteln, da die Änderung des Abstandes der
distalen Löcher vom proximalen Nagelende aufgrund
der erfolgten Biegung vernachlässigbar gering ist.
Hierzu sind Zielgeräte bekannt, die über magnetische
Koppelung die seitliche Ablage der Nagelachse
gegenüber der Bohrrichtung ermitteln und kompensieren.
Dabei ist das Tiefenmaß der einzubringenden Bohrung
unerheblich, da die durch die Nagelbiegung verursachten Winkelfehler der Lochachse gegenüber der
Bohrachse nur gering sind. Ein solches Zielgerät
ist jedoch allein bei Nägeln mit kreisförmigem
Querschnitt einsetzbar. Nägel dieser Art können
sich aber nur in sehr geringem Maß an die Form des
Markraumkanals anpassen und verringern damit nicht
unerheblich die Verbundfestigkeit des genagelten
Knochens.

3. Eine weitere Möglichkeit besteht darin, noch steifere
Nägel als die unter Ziffer 2 erwähnten, aber ebenfalls mit kreisförmigem Querschnitt, zu verwenden.
Dies hätte den Vorteil, daß ein nach dem unter Ziffer
1 genannten Verfahren arbeitendes Zielgerät eingesetzt werden könnte, weil hierbei auch eine Verbiegung des Nagels beim Eintreiben vermieden werden
kann. In diesem Fall sind jedoch die unter Ziffer 2
genannten Nachteile noch stärker ausgeprägt. Hinzu
kommt jedoch noch der Nachteil, daß in diesem Fall
der Markraumkanal in weit stärkerem Ausmaß vor
Einbringung des Marknagels aufzubohren ist, was
nicht nur zu den erwähnten Nachteilen führt, sondern
auch eine stärkere Läsion der Knochensubstanz mit
sich bringt.

4. Um diese genannten Nachteile zu vermeiden, ist es die am weitesten verbreitete Praxis, daß die gewünschten Nägel, gleich welcher Art, zunächst eingetrieben werden und daß anschließend die distalen Löcher mit Hilfe eines Röntgengerätes - im allgemeinen als Bildwandler bezeichnet - aufgesucht werden. Hierbei ist die angenähert punktförmige Strahlenquelle genau in die Richtung der verlängerten Achse des gesuchten distalen Loches zu bringen, was anhand der Schattenbildung der Lochränder erkennbar ist, da sich die kreisförmige Bohrung nur dann auf dem Bildschirm auch als Kreis darstellt, wenn diese Position erreicht ist. Es existieren bereits Zielgeräte, die mit dem Bildwandler dergestalt mechanisch verbunden sind, daß an ihnen nach dem Auffinden der richtigen Position eine Bohrlehre angebracht werden kann, deren Achse mit der Achse der Bohrung fluchtet. Die genaue Ausrichtung des großen und schweren Bildwandlers bereitet jedoch Mühe und ist umständlich, auch die Fixierung des Bildwandlers relativ zu Nagel und Knochen bereitet Schwierigkeiten. Ein erheblicher Nachteil bei diesen bekannten Geräten ist aber darüberhinaus die Strahlenbelastung des medizinischen Personals wie des Patienten beim Ausrichten und bei der Fixation der Bohrlehre. Schließlich ist auch das Einbringen der Löcher in den Knochen mittels dafür vorgesehener Bohrmaschinen dabei ein unbequemes und unsicheres Verfahren.

5. Zielgeräte der zuletzt beschriebenen Art kommen daher in praxi selten zur Anwendung, vielmehr wird der Durchstoßpunkt der Bohrung durch die Hautoberfläche mittels einer in den Strahlengang des Bildwandlers gebrachten Skalpellspitze markiert, danach wird der Bildwandler aus dem Operationsbereich weggefahren. Anschließend wird das Skalpell zur Inzision der Weichteile benutzt, in diese wird eine Bohrlehre eingebracht, und dann wird nach Augenmaß die Bohrung in den Knochen eingebracht. Dieses am weitesten verbreitete Verfahren erhöht jedoch gleichzeitig gegenüber dem vorher unter Ziffer 4 angeführten Ver-Verfahren die Strahlenbelastung des Operateurs sowie wesentlich auch die Gefahr einer Fehlbohrung. Fehlbohrungen müssen dann in Kauf genommen werden, ein Nachbohren oder Nachrichten ist nicht möglich und als unausweichliche Folge gelangen Metallspäne des Nagelmaterials in die Markraumhöhle. Dabei ist auch ein einwandfreier Sitz der Querbolzen mit der davon abhängenden ordnungsgemäßen Fixierung der Knochenfragmente schwerlich erzielbar.

Aufgabe der Erfindung ist es, eine Vorrichtung zum Auffinden von Querbohrungen intramedullärer Implantate, insbesondere der distalen Löcher von Verriegelungsnägeln zu schaffen, die ein schnelles, bequemes und sicheres Ansetzen der Bohrlehre in axialer Flucht zu den Querbohrungen gestattet, ohne daß eine Strahlenbelastung von Chirug und medizinischem Personal auftritt.

Erfindungsgemäß wird diese Aufgabe dadurch gelöst, daß zumindest ein erstes Richtelement in Form eines ein Richtfeld erzeugenden Magneten und ein zweites Richtelement in Form einer Magnetfeldmeßeinrichtung mit gerichteter Empfangscharakteristik vorgesehen sind, daß eines der beiden Richtelemente mit einer ausgezeichneten Empfangsrichtung der Achse der zu lokalisierenden Querbohrung ausrichtbar ist, daß das andere Richtelement frei beweglich ist und daß die Ausgangssignale der Magnetfeldmeßeinrichtung über eine Signalauswerteeinrichtung einer die Relativstellung der Richtcharakteristika beider Richtelemente zueinander darstellenden Anzeigevorrichtung zugeführt sind. Bevorzugt ist das magnetische Richtfeld axial- bzw. rotationssymmetrisch ausgerichtet.

Bei der erfindungsgemäßen Vorrichtung werden also die Richtcharakteristika von Magnetfeld und Magnetfeldeinrichtung dazu benutzt, die Relativstellung der Achsen beider Richtelemente über eine Anzeigeeinrichtung zu kontrollieren, so daß anhand dieser Kontrollanzeige beide Achsen zueinander in Kongruenz gebracht werden können. Das eine der beiden Richtelemente wird, beispielsweise durch mechanische Mittel im Inneren des Implantats, genau zur Achse der Querbohrung ausgerichtet, während das andere, außerhalb befindliche Richtelement zur Markierung der aufgefundenen Bohrung, etwa auf der Haut, oder zur Positionierung einer Bohrlehre benutzt werden kann.

Zur Verringerung von Meßfehlern kann der Suchvorgang durch zwei unabhängige Meßvorgänge erfolgen, bei denen zwei Punkte der Achse einer Implantatquerbohrung aufgefunden werden, die eindeutig die Lage dieser Achse bestimmen. Dabei ist es zweckmäßig, daß etwa zwei unterschiedliche bewegliche Richtelemente vorgesehen werden, von denen eines beispielsweise zur Auffindung eines Punktes auf der Körperoberfläche und ein zweites zur Auffindung eines weiteren Punktes unmittelbar auf der Knochenoberfläche geeignet ist.

Die beiden Richtelemente können wahlweise so gestaltet und angeordnet werden, daß in einem Fall die Magnetfeldmeßeinrichtung mit der Achse der Querbohrung im Implantat verknüpft wird und der Magnet außerhalb beweglich angeordnet ist und daß im anderen Fall die Achse der Querbohrung im Implantat mit dem Magnetfeld bzw. dem Magneten verknüpft wird und die Magnetfeldmeßeinrichtung außerhalb beweglich angeordnet ist.

Zur Erzeugung des Magnetfeldes kann beispielsweise ein Permanentmagnet oder auch ein Elektromagnet Verwendung finden. Die Magnetfeldmeßeinrichtung wird

zweckmäßigerweise mit einem oder mehreren magnetisch steuerbaren Halbleiterelementen versehen, wobei die Verwendung von Hallelementen besonders vorteilhaft ist. Es können aber auch Feldplatten oder andere Elemente mit vergleichbaren Effekten verwendet werden.

Die Magnetfeldmeßeinrichtung kann in der Weise angeordnet werden, daß das Maximum ihrer Meßempfindlichkeit jeweils mit der mit seiner Richtcharakteristik verknüpften Achse zusammenfällt, so daß diese durch das Aufsuchen des Signalmaximums bestimmbar ist. Bei einer anderen vorteilhaften Ausführungsform ist die Magnetfeldmeßeinrichtung in der Weise mit der Achse des betreffenden Richtelementes verknüpft, daß diese Achse mit dem Minimum ihrer Richtcharakteristik zusammenfällt, so daß die Lage dieser Achse durch Auffinden des Signalminimums bestimmbar ist. Wie erwähnt, können beispielsweise zwei Suchpunkte auf der Achse der Querbohrung des Implantats nacheinander bestimmt werden, wobei der erste der Durchstoßpunkt dieser Achse durch die Hautoberfläche ist und gleichzeitig die Eindringstelle für die notwendige Stichinzision durch die Weichteile bis zum Knochen markiert und wobei der zweite Suchpunkt nach erfolgter Stichinzision auf der Knochenoberfläche bestimmt wird. So kann zweckmäßigerweise zur Erzeugung des Magnetfeldes ein Ringmagnet verwendet werden, der eine leichte Markierung des ersten Suchpunktes auf der Hautoberfläche ermöglicht. Nach Einführung einer Bohrlehre in die Stichinzision kann in diese Bohrlehre ein anderer Magnet in Form einer Magnetsonde eingeführt werden, die es gestattet, den zweiten Suchpunkt auf der Knochenoberfläche präzise zu bestimmen.

Die Ausrichtung des einen Richtelementes in der Achse der Querbohrung im Implantat kann in zweckmäßiger Ausgestaltung dadurch ermöglicht werden, daß eines der Richtelemente am Kopf einer in das ein Hohlprofil aufweisende Implantat einführbaren Sonde befestigt ist, wobei der Sondenkopf in reproduzierbarer Entfernung von dem zugänglichen Ende des Implantats positionierbar ist. Die Sonde kann dabei einen einstellbaren Anschlag zur Positionierung des Sondenkopfs entsprechend der unterschiedlichen, im Einzelfall jedoch jeweils bekannten Entfernung der Querbohrung vom zugänglichen Ende des Implantats aufweisen.

Eine mögliche Winkelabweichung der Richtcharakteristik der Sonde gegenüber der Achse der Querbohrung im Implantat kann dadurch verhindert werden, daß der Sondenkopf dem Innenprofil des Implantats angepaßt ist und in diesem als Führung mit seiner Richtungscharakteristik koaxial zur Querbohrung positionierbar ist.

Die Signalauswerteeinrichtung ist zweckmäßigerweise mit einer Einrichtung für einen Nullabgleich versehen, um magnetische Störfelder, wie beispielsweise das Erdmagnetfeld, vor Beginn der Messung zu kompensieren. Als Anzeigeeinrichtung wird in den meisten Fällen ein optisches Anzeigegerät, beispielsweise der in Operationssälen ohnehin verfügbare Monitor eines Bildwandlers eingesetzt werden. Daneben sind aber auch andere optische Anzeigeeinrichtungen , aber auch akustische bzw. auf sonstige Sinnesorgane einwirkende Signalgeräte anwendbar.

Die Erfindung wird nachfolgend an einem Ausführungsbeispiel anhand der Zeichnung näher erläutert. Es zeigt:

Fig. 1  die Anordnung einer erfindungsgemäßen Vorrichtung an einem implantierten Verriegelungsnagel

Fig. 2 einen Querschnitt durch einen Verriegelungsnagel im Bereich einer Querbohrung mit eingesetzter Magnetfeldmeßeinrichtung und

Fig. 3 eine Seitenansicht des Verriegelungsnagels in Axialrichtung der Querbohrung mit eingesetzter Magnetfeldmeßeinrichtung.

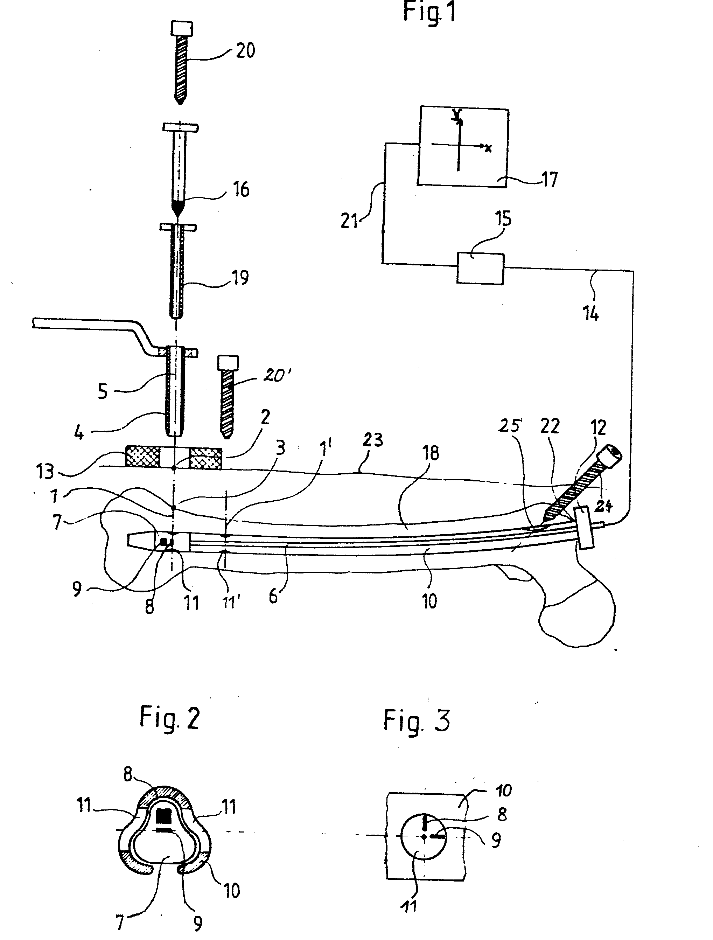

Fig. 1 zeigt die Anordnung eines Verriegelungsnagels 10 im Markraum eines Knochens 18, der in einem bestimmten Abstand unterhalb der Haut bzw. Körperoberfläche 23 liegt. Der Nagel ist in Fig. 1 von rechts in den Knochen eingetrieben und besitzt in der Nähe seines linken, d.h. distalen Endes zwei Querbohrungen 11 und 11' zur Aufnahme von Querbolzen 20 bzw. 20'.

Es soll nun die genaue Lage der jeweiligen Bohrachse 1 eines distalen Loches 11 im Verriegelungsnagel 18 ermittelt werden. Diese Bohrachse ist eindeutig bestimmbar durch die geradlinige Verbindung von zwei Punkten 2 und 3 auf dieser Achse. Um eine Bohrlehre 4 in eine damit fluchtende Position zu bringen, sind demnach die zwei Punkte 2 und 3 auf der Bohrungsachse des distalen Loches 11 aufzufinden, und die Achse der Bohrlehre ist nach diesen Punkten auszurichten, wonach sofort die Bohrung in den Knochen eingebracht werden kann. Der erste dieser beiden Punkte 2 wird an der Hautoberfläche 23 ermittelt und gibt damit gleichzeitig den Ort der Stichinzision an. Der zweite Punkt 3 wird an der Oberfläche der Außenkortikalis bestimmt und liefert damit gleichzeitig den Punkt am Knochen, durch den der Bohrer einzudringen hat. Dabei nimmt die durch den ersten Punkt 2 geführte und bis zum Knochen reichende Stichinzision gleichzeitig die Bohrlehre 4 auf, die damit ihre Achse 5 durch den zuerst gefundenen Punkt 2 führt.

Zur Auffindung dieser beiden Punkte 2 und 3 dient die erfindungsgemäße Vorrichtung, welche eine Magnetfeldmeßeinrichtung in Gestalt einer Sonde 6 mit einem Sondenkopf 7 und darauf angebrachten Hallelementen 8 und 9 aufweist. Die beiden Hallelemente 8 und 9 sind dabei so angebracht, daß sich ihre Hauptebenen in der Achse 1 der distalen Bohrung 11 schneiden. Diese Achse 1 ist der geometrische Ort aller Punkte, an denen ein axialsymmetrisch angelegtes Magnetfeld keine Ausgangssignale an den Hallelementen erzeugt. Dies wird dadurch erreicht, daß der Sondenkopf 7 gemäß Fig. 2 als Formstück entsprechend dem Innenprofil des Nagels 10 ausgeführt ist, dieser Sondenkopf wird somit im Inneren des hier kleeblattförmigen Profils des Nagels 10 beim Einführen der Sonde 6 geführt, selbst wenn der Nagel 10 beim Eintreiben eine wie auch immer realistisch geartete Verbiegung und/oder Verwindung erfahren hat. In Längsrichtung des Nagels 10 wird die genaue Ausrichtung des Sondenkopfes 7 bzw. der Hallgeneratoren 8 und 9 zur Achse der Querbohrung 11 damit erreicht, daß der Abstand der Querbohrung 11 vom proximalen Ende 22 des Nagels 10 bei realistischer Ausprägung der Deformation und Berücksichtigung der elastischen Verformbarkeit der Sonde 6 in ihrem Schaft mit ausreichender Genauigkeit erhalten bleibt. Für verschiedene Nagellängen bzw. für die verschiedenen distalen Löcher 11 oder 11' des Nagels 10 kann ein Anschlag 12 auf dem Sondenschaft 6 an entsprechend unterschiedlichen Stellen vor dem Einbringen des Nagels in den Knochen positioniert und fixiert werden. Für unterschiedliche Nageldurchmesser bzw. unterschiedliche Nagelprofilformen sind entsprechend unterschiedlich ausgebildete Sondenköpfe 7 notwendig.

Zu der dargestellten Vorrichtung gehört weiter ein Suchmagnet in Form eines Ringmagneten 13 sowie ein Stabmagnet 16. Zum Einbringen der Bohrung dient die bereits erwähnte Bohrlehre 4 mit einem zusätzlichen Einsatz 19.

Die Ausgangssignale der Hallelemente 8 und 9 sind über eine Leitung 14 zu einer Signalverarbeitungseinrichtung 15 geführt, von der aus auch die Hallelemente in üblicher Weise elektrisch versorgt werden. Die Ausgangssignale der Verarbeitungseinheit 15 werden über eine Leitung 21 einem Anzeigegerät 17 zugeführt, auf welchem dann jeweils die relative Stellung der Achsen der Magnetfeldmeßeinrichtung einerseits und des gerade verwendeten Magneten 13 oder 16 andererseits sichtbar gemacht wird.

Vor Beginn der Messung ist ein Abgleich der Anzeige erforderlich. Zu diesem Zweck müssen die Suchmagnete 13 und 16 von der Anordnung entfernt gehalten werden, so daß der Nullpunkt der Anzeigeeinheit 17 abgeglichen werden kann, um dabei Einflüsse des Erdmagnetfeldes und anderer stationärer Störfelder zu kompensieren. Zum eigentlichen Suchvorgang wird nun der Ringmagnet 13 in die Nähe der Hallelemente 8 und 9 gebracht und auf der Hautoberfläche 23 verschoben. Die Anzeige weicht von dem beim Nullabgleich eingestellten Wert so lange ab, als das am Ort der Hallelemente aufgebrachte Magnetfeld des Ringmagneten 13 so gerichtet ist, daß es einen dort nicht parallel zu beiden Hallelementen gerichteten Feldstärkevektor aufweist.

Bei geeigneter Magnetisierung des Ringmagneten 13 sind dessen Feldlinien in der nahen Umgebung seiner Rotationsachse ausreichend geradlinig und parallel zu ihr. Bei annähernd senkrechter Anordnung der Rotationsachse des Ringmagneten 13 zu der Längsachse des Nagels 10 im Bereich des Sondenkopfes 7 erreichen die Ausgangssignale der beiden Hallelemente 8 und 9 nur dann wieder den bei Nullabgleich der Signalverarbeitungseinrichtung 15 eingestellten Wert, wenn die Rotationsachse des Ringmagneten 13 und die Achse der Bohrung 11 mit ausreichender Genauigkeit miteinander fluchten. Damit ist der Durchstoßpunkt der Rotationsachse des Ringmagneten 13 durch die Hautoberfläche 23 des Patienten gleichzeitig der erste zu ermittelnde Punkt 2. Er kann als Mittelpunkt der Öffnung des Ringmagneten 13 festgehalten und auf der Hautoberfläche markiert werden.

Die nicht vollständig geradlinige Gestalt der Magnetlinien des Ringmagneten 13 außerhalb seiner Rotationsachse ermöglicht bei dem genannten Vorgehen einen Winkelfehler, dessen Tangens am Ort des Knochens zu einer Fehlbohrung führen könnte, wenn die Bohrlehre 4 allein nach der Lage des Ringmagneten 13 bei Erreichung des eingestellten Nullwertes vorgenommen werden würde.

Aus diesem Grund ist bei der Vorrichtung ein zweiter Magnet 16 in Form einer stiftförmigen Magnetsonde vorgesehen, mit der ein zweiter Suchvorgang durchgeführt werden kann. Dazu wird nach erfolgter Stichinzision in die ohnehin erforderliche Öffnung durch die den Knochen umschließenden Weichteile die Bohrlehre 4 eingeführt, wobei das dem Knochen abgewandte Ende dieser Bohrlehre im Punkt 2 durch den nach dem ersten Suchvorgang festgelegten Hautschnitt ausreichend genau gehalten wird. Das dem Knochen zugewandte Ende der Bohrlehre 4 ist aufgrund einer gewissen Nachgiebigkeit der Weichteile in geringem Ausmaß tangential auf der Knochenoberfläche verschiebbar.

In diese Bohrlehre 4 wird nunmehr die Magnetsonde 16 eingeführt, welche in ihrer Formgebung an den Innendurchmesser der Bohrlehre angepaßt ist und so magnetisiert ist, daß sie ein Magnetfeld erzeugt, dessen Feldlinien im Achsenbereich ausreichend genau geradlinig und parallel zu dieser Achse verlaufen. Der Suchvorgang geht prinzipiell wie der vorher beschriebene erste Suchvorgang vor sich, wobei das dem Knochen zugewandte Ende der Bohrlehre durch geringfügige Verschiebung auf der Knochenoberfläche in eine ausreichend genaue Position zentral zur betrachteten Bohrung 11 im Nagel 10 gebracht wird. Das dem Knochen abgewandte Ende der Bohrlehre wird dabei mit ausreichender Genauigkeit im vorher festgelegten Suchpunkt 2 gehalten.

In dieser Stellung der Bohrlehre 4 kann nach Entfernen der Magnetsonde 16 der Bohreinsatz 19 eingeführt werden, während gleichzeitig die Hallsonde 6 aus dem Nagel 10 entfernt wird. Dann kann der Knochen 18 durch den Einsatz 19 vorgebohrt werden, anschließend wird der Einsatz 19 wieder entfernt und die der Bohrlehre 4 zugewandte Kortikalis des Knochens 18 mit größerem Durchmesser zur Aufnahme des Bolzenschaftes des Bolzens 20 aufgebohrt. Die zuerst unter Verwendung des Einsatzes 19 auch in die Gegenkortikalis eingebrachte Bohrung verbleibt zur Aufnahme des Gewindes des Bolzens 20 in ihrem vorherigen Durchmesser. Anschließend werden Bohrer und Bohrlehre entfernt, und die Verriegelungsbolzen 20 werden in die Bohrung gesetzt.

Vor den beschriebenen Arbeiten am distalen Ende wird der proximale Bolzen 24 in die entsprechend Bohrung 25 gesetzt, was hier nicht näher beschrieben wird, weil für das Auffinden der proximalen Löcher wegen der geringen Distanz zum proximalen Nagelende mit einer herkömmlichen Ziel- und Bohrvorrichtung gemäß der Beschreibung unter Ziffer 1 der Einleitung ausreichend präzise gearbeitet werden kann.

Bei Anwendung der erfindungsgemäßen Vorrichtung können somit die gewohnten Implantate und das herkömmliche Instrumentarium für die Arbeiten am proximalen Bereich weiterhin verwendet werden. Dies gilt prinzipiell auch für die Arbeiten im distalen Bereich.
So kann das Implantatmaterial des Nagels 10 und der Bolzen 20 in herkömmlicher Ausführung verwendet werden, desgleichen die Bohrlehre 5 und der Einsatz 19. Die erfindungsgemäße Zielvorrichtung mit der Sonde 6 und dem Sondenkopf 7, den beiden Magneten 13 und 16 sowie mit der Auswerteeinrichtung 15 und der Anzeigeeinrichtung 17 kann zusätzlich zu dem herkömmlichen Instrumentarium verwendet werden. Bei den herkömmlichen Geräten ist lediglich darauf zu achten, daß die Bohrlehre 4 und der Einsatz 19 aus nicht magnetischem Material gefertigt sein müssen. Diese Vorraussetzung ist bei den Implantaten ohnehin aufgrund von Vorschriften für die Implantatwerkstoffe gegeben.

Ergänzend sei noch erwähnt, daß die Vorrichtung auch dann wirksam arbeiten kann, wenn eine Führung des Sondenkopfes durch das Innenprofil des Implantats nicht möglich ist, z.B.bei Nägeln mit kreisrundem Querschnitt. In diesem Fall können zusätzliche Maßnahmen vorgesehen werden, um den Sondenkopf zur Querbohrung auszurichten. Beispielsweise kann dies mittels einer in der Bohrung einrastbaren Haltevorrichtung geschehen.

0135804

Patentansprüche:

1.    Vorrichtung zum Auffinden von Querbohrungen intramedullärer Implantate bei der Frakturbehandlung von Knochen, insbesondere zum Auffinden von Querbohrungen am
distalen Ende von implantierten Verriegelungsnägeln, und
zur koaxialen Ausrichtung einer Bohrvorrichtung zu diesen
Querbohrungen, dadurch gekennzeichnet, daß zumindest ein
erstes Richtelement in Form eines ein Richtfeld erzeugenden Magneten (13,16) und ein zweites Richtelement in Form
einer Magnetfeldmeßeinrichtung (8,9) mit gerichteter Empfangscharakteristik vorgesehen sind, daß eines der beiden
Richtelemente mit einer ausgezeichneten Empfangsrichtung
der Achse der zu lokalisierenden Querbohrung (11) ausrichtbar ist, daß das andere Richtelement frei beweglich
ist und daß die Ausgangssignale der Magnetfeldmeßeinrichtung (8,9) über eine Signalauswerteeinrichtung (15) einer
die Relativstellung der Richtcharakteristika beider Richtelemente zueinander darstellenden Anzeigevorrichtung (17)
zugeführt sind.

2.    Vorrichtung nach Anspruch 1, dadurch gekennzeichnet,
daß mindestens zwei an unterschiedlichen Körperstellen anbringbare frei bewegliche Richtelemente (13,16) vorgesehen
sind.

3.    Vorrichtung nach Anspruch 1 oder 2, dadurch gekennzeichnet, daß die Magnetfeldmeßeinrichtung (8,9) in der
Achse der Querbohrung (11) des Implantats (10) ausrichtbar und daß der Magnet (13,16) frei beweglich ist.

4.    Vorrichtung nach Anspruch 1 oder 2, dadurch gekennzeichnet, daß der Magnet in der Achse der Querbohrung ausrichtbar ist und daß die Magnetfeldmeßeinrichtung frei beweglich ist.

2

0135804

5. Vorrichtung nach einem der Ansprüche 1 bis 4, dadurch gekennzeichnet, daß das erste Richtelement ein Permanentmagnet (13, 16) ist.

6. Vorrichtung nach einem der Ansprüche 1 bis 4, dadurch gekennzeichnet, daß das erste Richtelement ein Elektromagnet ist.

7. Vorrichtung nach einem der Ansprüche 1 bis 6, dadurch gekennzeichnet, daß die Magnetfeldmeßeinrichtung einen oder mehrere magnetisch steuerbare Halbleiterelemente (8, 9) enthält.

8. Vorrichtung nach Anspruch 7, dadurch gekennzeichnet, daß die Magnetfeldmeßeinrichtung einen oder mehrere Hallgeneratoren (8, 9) enthält.

9. Vorrichtung nach einem der Ansprüche 1 bis 8, dadurch gekennzeichnet, daß die Magnetfeldmeßeinrichtung das Maximum ihrer Meßempfindlichkeit in der Achse ihrer Richtcharakteristik besitzt.

10. Vorrichtung nach einem der Ansprüche 1 bis 8, dadurch gekennzeichnet, daß die Richtachse der Magnetfeldmeßeinrichtung mit dem Minimum ihrer Empfangscharakteristik zusammenfällt.

11. Vorrichtung nach einem der Ansprüche 1 bis 8 sowie 10, dadurch gekennzeichnet, daß in der Magnetfeldmeßeinrichtung zwei Hallgeneratoren (8, 9) zueinander im Winkel derart ausgerichtet sind, daß ihre beiden Hauptebenen sich in der Richtachse der Magnetfeldmeßeinrichtung schneiden.

12. Vorrichtung nach Anspruch 11, dadurch gekennzeichnet, daß die beiden Hallgeneratoren (8, 9) zueinander im rechten Winkel stehen.

13. Vorrichtung nach einem der Ansprüche 1 bis 12, dadurch gekennzeichnet, daß als erstes Richtelement ein Ringmagnet (13) dient.

14. Vorrichtung nach einem der Ansprüche 1 bis 12, dadurch gekennzeichnet, daß als erstes Richtelement eine in eine Bohrlehre (4) einführbare Magnetsonde (16) dient.

15. Vorrichtung nach einem der Ansprüche 1 bis 14, dadurch gekennzeichnet, daß eines der Richtelemente (8, 9) am Kopf (7) einer in das ein Hohlprofil aufweisende Implantat (10) einführbaren Sonde (6) befestigt ist, wobei der Sondenkopf (7) in reproduzierbarer Entfernung von dem zugänglichen Ende (22) des Implantats (10) positionierbar ist.

16. Vorrichtung nach Anspruch 15, dadurch gekennzeichnet, daß die Sonde (6) einen einstellbaren Anschlag (12) zur unterschiedlichen Positionierung des Sondenkopfes (7) aufweist.

17. Vorrichtung nach einem der Ansprüche 1 bis 16, dadurch gekennzeichnet, daß der Sondenkopf (7) dem Innenprofil des Implantats (10) angepaßt ist und in dieser Ausführung mit seiner Richtcharakteristik koaxial zur Querbohrung (11) positionierbar ist.

18. Vorrichtung nach einem der Ansprüche 1 bis 17, dadurch gekennzeichnet, daß in der Signalauswerteeinrichtung (15) eine Abgleicheinrichtung zur Kompensation von magnetischen Störfeldern vorgesehen ist.

19. Vorrichtung nach einem der Ansprüche 1 bis 18, dadurch gekennzeichnet, daß die Signalauswerteeinrichtung (15) die Ausgangssignale zur Darstellung in einer optischen Anzeigeeinrichtung (17) umsetzt.

20. Vorrichtung nach Anspruch 19, dadurch gekennzeichnet, daß als Anzeigeeinrichtung ein üblicher Monitor verwendbar ist.

21. Vorrichtung nach einem der Ansprüche 1 bis 18, dadurch gekennzeichnet, daß als Anzeigeeinrichtung eine auf das Gehör oder ein anderes menschliches Sinnesorgan einwirkende Signaleinrichtung vorgesehen ist.

22. Vorrichtung nach einem der vorstehenden Ansprüche, dadurch gekennzeichnet, daß das Richtfeld des Magneten (13,16) ein axial- bzw. rotationssymmetrisches Magnetfeld ist.

0135804

1/1

Fig. 1

Fig. 2

Fig. 3